(19) Europäisches Patentamt
European Patent Office
Office européen des brevets

(11) Veröffentlichungsnummer: **0 105 494**
**B1**

(12) # EUROPÄISCHE PATENTSCHRIFT

(45) Veröffentlichungstag der Patentschrift: **10.08.88**

(51) Int. Cl.⁴: **C 07 D 277/68**

(21) Anmeldenummer: **83109804.1**

(22) Anmeldetag: **30.09.83**

(54) **Verfahren zur Herstellung von Benzthiazolyloxyphenoxypropionsäurederivaten.**

(30) Priorität: **04.10.82 DE 3236730**

(43) Veröffentlichungstag der Anmeldung: **18.04.84 Patentblatt 84/16**

(45) Bekanntmachung des Hinweises auf die Patenterteilung: **10.08.88 Patentblatt 88/32**

(84) Benannte Vertragsstaaten: **CH DE FR GB IT LI NL**

(56) Entgegenhaltungen:
**FR-A-2 440 352**
**GB-A-2 046 753**
**US-A-4 130 413**

(73) Patentinhaber: **HOECHST AKTIENGESELLSCHAFT, Postfach 80 03 20, D-6230 Frankfurt am Main 80 (DE)**

(72) Erfinder: **Zeiss, Hans- Joachim, Dr., Ziegenhainer Weg 12, D-3578 Schwalmstadt (DE)**
Erfinder: **Mildenberger, Hilmar, Dr., Fasanenstrasse 24, D-6233 Kelkheim (Taunus) (DE)**
Erfinder: **Handte, Reinhard, Dr., Breckenheimer Strasse 45, D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 105 494 B1**

LIBER, STOCKHOLM 1988

**Beschreibung**

Gegenstand der vorliegenden Erfindung ist ein Verfahren zur Herstellung von Verbindungen der Formel I

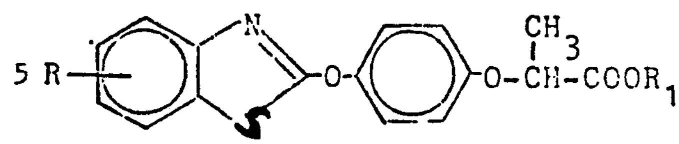

worin

R: Halogen oder $CF_3$ und

$R_1$: ($C_1$-$C_{12}$)Alkyl, das durch ein- oder zwei Halogenatome oder eine ($C_1$-$C_6$)Alkoxy-, ($C_1$-$C_6$)Alkylthio-, ($C_1$-$C_6$)Alkoxy-($C_2$-$C_4$)-alkoxy, Halogen-($C_1$ oder $C_2$)alkoxy- oder Methoxyethoxyethoxygruppe substituiert sein kann; ($C_3$-$C_6$)Alkonyl, ($C_3$-$C_6$) Alkinyl, ($C_5$ oder $C_6$)Cycloalkyl, ($C_1$-$C_4$)Alkoxycarbonyl-($C_1$ oder $C_2$)alkyl oder die Gruppe $-N=C[(C_1$-$C_4)$-Alkyl$]_2$ bedeuten.

Verbindungen der Formel (I) sind z. B. aus US-B-4 130 413, GB-A-20 46 753 und EP-2 800 bekannt; sie zeichnen sich durch selektive Herbizidwirkung gegen Ungräser aus. Bezüglich der einzelnen Bedeutungen von $R_1$ wird insbesondere auf die darin beschriebenen Verbindungen verwiesen. Bevorzugt sind vor allem die ($C_1$-$C_{12}$)-Alkylreste. Unter Halogen wird bevorzugt Fluor, Chlor und Brom verstanden.

Die Herstellung der Verbindungen erfolgt gemäß dem genannten Stand der Technik durch Umsetzung von 2-Halogenbenzthiazolen der Formel II mit substituierten Phenolen der Formel III in Gegenwart von Säurebindern:

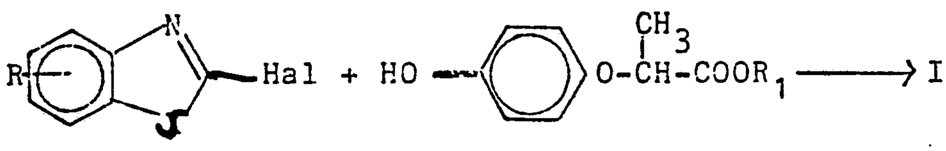

II III

Zur Umsetzung verwendet man inerte aprotische Lösungsmittel wie aliphatische oder aromatische Kohlenwasserstoffe (Benzol, Toluol, Xylol), Säurenitrile (Acetonitril), Ketone (Aceton) oder Säureamide (DMF, DMSO). Aus dem einen oder anderen Grund weisen diese Lösungsmittel jedoch Nachteile auf. DMF, DMSO und Acetonitril, die an sich zu hohen Ausbeuten führen, sind ziemlich teuer und wegen ihrer relativ niedrigen MAK-Werte aus gewebehygienischer Sicht nicht ganz unbedenklich. Ketone bringen wegen ihrer niedrigen Siedepunkte keine optimalen Ausbeuten. Unpolare Lösungsmittel wie Toluol und Xylol wiederum führen trotz extrem langer Reaktionszeiten zu Produkten mit ungenügender Reinheit in ebenfalls unbefriedigenden Ausbeuten.

Die Aufgabe der vorliegenden Erfindung bestand nun darin, ein Verfahren zu finden, bei dem die zuvor beschriebenen Nachteile vermieden werden.

Überraschenderweise wurde gefunden, daß die Verbindungen II und III in unpolaren Lösungsmitteln in hohen Ausbeuten zu den Verbindungen I umgesetzt werden können, wenn in Gegenwart eines Katalysators gearbeitet wird.

Gegenstand der Erfindung ist daher ein Verfahren zur Herstellung von Verbindungen der Formel I durch Umsetzung von Verbindungen der Formel II mit Verbindungen der Formel III in Gegenwart eines inerten unpolaren Lösungsmittels und eines säurebindenden Mittels, welches dadurch gekennzeichnet ist, daß die Reaktion in Gegenwart eines Katalysators aus der Gruppe der quartären Ammonium- und Phosphoniumbasen, der Polyalkylenglycole oder deren Niederalkylether durchgeführt wird.

Die Katalysatoren werden in Mengen von 0,05 bis 1,0 mol %, bevorzugt 0,1 bis 0,5 mol %, bezogen auf die Komponente II angewendet. Die quartären Ammonium- und Phosphoniumbasen enthalten als organische Reste Alkyl, Benzyl oder Phenylgruppen und liegen üblicherweise als Halogenide vor. Als Beispiele seien genannt:

Tetrabutylammoniumbromid, Tetrabutylammoniumjodid, Triethylbenzylammoniumchlorid, Tetraoctylphosphoniumchlorid, Tetrabutylphosphoniumbromid, Triphenylbutylphosphoniumbromid, Trilaurylmethylphosphoniumjodid, Tributylmethylphosphoniumjodid und Tributylhexadecylphosphoniumbromid. Daneben eigenen sich cyclische Polyethylenglycole (Kronenether) und langkettige Polypropylen- und besonders Polyethylenglykole mit Molekulargewichten von ca. 400 - ca. 6 000 sowie deren Mono- und Diniederalkylether. Verbindungen dieser Art sind in der organischen Chemie allgemein unter der Bezeichnung "Phasentransferkatalysatoren" bekannt, da sie Reaktionen im Zweiphasensystem Wasser/org. Lösungsmittel beschleunigen.

Vorzugsweise werden wegen der besseren Phasentrennung bei der wäßrigen Aufbereitung langkettige oder cyclische Polyalkylenglykole mit Molekulargewichten zwischen ca. 1 000 und ca. 3 000 eingesetzt.

Als Lösungsmittel verwendet man bei Raumtemperatur flüssige aromatische Kohlenwasserstoffe, die auch halogenhaltig sein können, wie z. B. Toluol, Xylol, Cumol, Mesitylen.

Als säurebindende Mittel kommen allgemein organische und anorganische Basen, bevorzugt jedoch Alkalicarbonate und -hydrogencarbonate, wie z. B. die des Kaliums und Natriums in Frage. Zur Vermeidung von Hydrolyse- bzw. Verseifungsreaktionen, die Ausbeuteminderung zur Folge haben, muß bei Verwendung der letzteren das Reaktionswasser entfernt werden. Es ist außerdem zu beachten, daß bei Temperaturen oberhalb von ca. 90°C durch thermische Instabilität aus den eingesetzten bzw. durch Neutralisation aus den Alkalicarbonaten gebildeten Hydrogencarbonaten Wasser entsteht. Das Wasser kann entweder azeotrop bei Normaldruck oder unter reduziertem Druck sowie mit Hilfe eines Inertgasstromes abdestilliert werden.

In diesem Zusammenhang sei auf Synthesis 1980, 921 verwiesen, wo die Umsetzung von 2-Chlor-pyridinen und 2-Chlor-chinolinen mit Phenolen im System Wasser/Toluol bzw. Benzol unter Zusatz von Phasentransferkatalysatoren beschrieben wird. Bei den erfindungsgemäßen Verfahren wirken die verwendeten Katalysatoren infolge Fehlens einer zweiten Flüssigphase nicht als Phasentransferkatalysatoren, sondern als Lösungsvermittler. Es war überraschend, daß die Katalysatoren auch in einem System flüssig-fest hervorragend wirksam sind.

Das erfindungsgemäße Verfahren wird im Temperaturbereich von 30°C bis 250°C, bevorzugt zwischen 50 und 180°C bzw. dem Siedepunkt des Lösungsmittels durchgeführt. Die Reihenfolge der Zugabe der Reaktionspartner ist nicht kritisch; das Verfahren wird im allgemeinen so durchgeführt, daß man das 2-Halogenbenzthiazol und die Base zusammen mit dem Katalysator im gewählten Lösungsmittel vorlegt und unter Reaktionsbedingungen die Komponente III zutropfen läßt. Der üblicherweise verwendete geringe Überschuß der Komponente III läßt sich leicht dadurch entfernen, daß nach erfolgter Umsetzung der Ansatz abgekühlt und mit verdünnter Alkalilauge behandelt wird. Nach Neutralisieren und Abdestillieren des Lösungsmittels, welches erneut verwendet werden kann, hinterbleiben die Verfahrenserzeugnisse in fast quantitativen Ausbeuten mit Reinheiten von ca. 95 % (gaschromatographisch bestimmt).

Die nachfolgenden Beispiele sollen das Verfahren näher erläutern, ohne daß dadurch eine Einschränkung beabsichtigt ist.

**Beispiel 1**

Ethyl-2-[4-(6-chlor-2-benzthiazolyloxy)-phenoxy]-propanoat

a) 105,0 g (0,5 mol) Ethyl-2-(4-hydroxyphenoxy)-propanoat, 102,0 g (0,5 mol) 2,6-Dichlorbenzthiazol, 70,5 g (0,51 mol) Kaliumcarbonat und 0,22 g (0,6 mmol) Tetra-n-butyl-phosphoniumbromid werden in 300 ml Xylol zusammengegeben und unter Stickstoffatmosphäre am Wasserabscheider 6,5 Stunden am Rückfluß gekocht. Der Fortgang der Reaktion wird dünnschichtchromatographisch verfolgt. Nach beendeter Umsetzung wird auf Raumtemperatur abgekühlt. Der Salzanteil wird abfiltriert, das Filtrat wird mit 200 ml Wasser gewaschen, die organische Phase wird eingeengt.

Es werden 183,0 g (96,8 % der Theorie) Ethyl-2-[4-(6-chlor-2-benzthiazolyloxy)-phenoxy]-propanoat vom Fp. 53,0 - 54,0°C erhalten. Die Reinheit beträgt $\geqslant$97 %.

b) 204 g (1 mol) 2,6-Dichlorbenzthiazol, 99,4 g (0,72 mol Kaliumcarbonat und 6 g (0,003 mol) Polyethylenglykol (MG 2000) werden in 600 ml Xylol vorgelegt und unter starkem Rühren auf 135 - 140°C erwärmt. Innerhalb einer Stunde werden 220,5 (1,05 mol) Ethyl-2-(4-hydroxyphenoxy)-propanoat unter Rückfluß und azeotropem Abdestillieren von Wasser zutropfen gelassen.

Unter weiterem Wasserauskreisen wird nach der Zugabe 7 h bei 140°C nachgerührt. Der Ansatz wird abgekühlt, einmal mit 400 ml und einmal mit 250 ml 5 %-iger Natronlauge gewaschen. Nach Neutralwaschen mit verdünnter Natriumdihydrogenphosphatlösung wird Xylol abdestilliert. Als Rückstand verbleiben 363,6 g (96,2 % der Theorie) Ethyl-2-[4-(6-chlor-2-benzthiazolyloxy)-phenoxy]-propanoat vom Fp 53,4 - 54,5°C; die Reinheit nach GC beträgt 97,5 %.

c) 255,0 g (1,25 mol) 2,6-Dichlorbenzthiazol, 124,4 g (0,9 mol) Kaliumcarbonat, 7,5 g (0,004 mol) Polyethylenglykol (MG 2000) und 750 ml Mesitylen werden unter starkem Rühren auf ca. 160°C erwärmt. Innerhalb einer Stunde werden 275,4 g (1,31 mol) Ethyl-2-(4-hydroxyphenoxy)-propanoat unter starkem Rückfluß tropfenweise zugegeben. Nach beendeter Zugabe wird noch 3,5 h unter Rückfluß weitergerührt, wobei sich bildendes Wasser ständig azeotrop abdestilliert wird. Der Ansatz wird abgekühlt, einmal mit 300 ml, einmal mit 200 ml 5 %-iger Natronlauge und anschließend mit Phosphatpuffer (pH 4) gewaschen. Die organische Phase wird abgetrennt, das Xylol abdestilliert. Es verbleiben 198,5 g (93,9 % der Theorie) an Ethyl-2-[4-(6-chlor-2-benzthiazolyloxy)-phenoxy]-propanoat. Die Reinheit beträgt 94,3 %.

d) 102,0 g (0,5 mol) 2,6-Dichlorbenzthiazol und 100,1 g (1 mol) Kaliumhydrogencarbonat werden in 200 ml Xylol auf Rückflußtemperatur erwärmt. Innerhalb einer Stunde wird unter Aufrechterhaltung eine kräftigen Rückflusses eine Lösung von 110,25 (0,525 mol) Ethyl-2-[4-hydroxyphenoxy]-propanoat und 3 g (0,0015 mol) Polyethylenglykol (MG 2000) in 100 ml Xylol zugetropft. Nach beendeter Zugabe wird noch 6,5 h unter Rückfluß weitergerührt, wobei sich bildendes Wasser ständig azeotrop abdestilliert wird. Die Reaktionsmischung wird abgekühlt, einmal mit 100 ml 5 %-iger Natronlauge und zweimal mit je 100 ml Wasser gewaschen. Nach dem

Abdestillieren des Xylols verbleiben 182,0 g (96,3 % der Theorie) Ethyl-2-[4-(6-chlor-2-benzthiazolyloxy)-phenoxy]-propanoat. Die Reinheit beträgt 93,6 %.

Analog Beispiel 1a erhält man:

| Beisp. Nr. | Katalysator | Ausbeute [%] | Reinheit [%] (lt. GC) |
|---|---|---|---|
| 2 | Ethyl-trioctylphosphoniumbromid | 97,4 | 95,9 |
| 3 | Trilaurylmethylphosphoniumbromid | 93,7 | 96,3 |
| 4 | Tetraoctylphosphoniumchlorid | 96,0 | 96,9 |
| 5 | Polyethylenglykol (MG 1500) | 94,2 | 96,9 |
| 6 | Polyethylenglykol (MG 3000) | 95,9 | 96,8 |
| 7 | Polypropylenglykol (MG 2000) | 94,9 | 94,9 |
| 8 | Polyethylenglykolmonomethylether (MG 1900) | 96,1 | 97,6 |
| 9 | Polyethylenglykoldimethylether (MG 400) | 96,2 | 98,2 |

MG = durchschnittliches Molekulargewicht

**Vergleichsbeispiele**

A) Ohne Katalysator, ohne Abdestillieren des bei der Reaktion sich bildenden Wassers (analog DE-OS-26 40 730).

115 g (0,55 mol) Ethyl-2-(4-hydroxyphenoxy)-propanoat, 102 g (0,5 mol) 2,6-Dichlorbenzthiazol und 70,4 g (0,51 mol) Kaliumcarbonat werden in 300 ml Xylol 30 h unter gutem Rühren am Rückfluß erhitzt. Der Ansatz wird abgekühlt, vom Salz befreit, zweimal mit je 250 ml 5 %-iger Natronlauge und anschließend mit Wasser neutral gewaschen. Nach dem Abdestillieren des Xylols verbleiben 65,8 g eines braunen Öls, welches gaschromatographisch zu 91,1 % aus Ethyl-2-[4-(6-chlor-2-benzthiazolyloxy)-phenoxy]-propanoat besteht, daneben sind noch 3,1 % unumgesetztes 2,6-Dichlorbenthiazol enthalten. Dies entspricht einer Ausbeute von nur 31,7 %.

B) ohne Katalysator, jedoch mit Abdestillieren des bei der Reaktion sich bildenden Wassers.

105,0 g (0,5 mol) Ethyl-2-(4-hydroxyphenoxy)-propanoat, 102,0 g (0,5 mol) 2,6-Dichlorbenzthiazol und 70,5 g (0,51 mol) Kaliumcarbonat werden in 300 ml Xylol zusammengegeben und unter Stickstoffatmosphäre am Wasserabscheider 19 Stunden am Rückfluß erhitzt. Danach wird die Reaktionsmischung wird auf Raumtemperatur abgekühlt, der Salzanteil abfiltriert, das Filtrat mit 200 ml Wasser gewaschen, die organische Phase abgetrennt und eingeengt. Lösungsmittelreste werden im Hochvakuum entfernt.

Es werden 162,3 g eines braunen Öls erhalten, daß laut gaschromatographischer Analyse zu 93,6 % aus Ethyl-2-[4-(6-chlor-2-benthiazolyloxy)-phenoxy]-propanoat und zu 6,4 % aus nichtumgesetztem 2,6-Dichlorbenthiazol besteht. Dies entspricht einer exakten Ausbeute von 80 %.

**Patentansprüche**

1. Verfahren zur Herstellung von Verbindungen der Formel I,

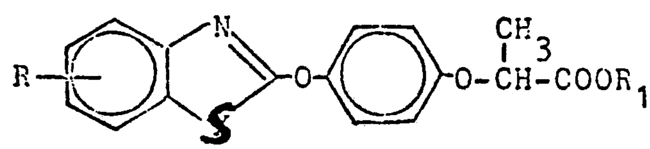

worin

R: Halogen oder $CF_3$ und

$R_1$: ($C_1$-$C_{12}$)Alkyl, das durch ein- oder zwei Halogenatome oder eine ($C_1$-$C_6$)Alkoxy-, ($C_1$-$C_6$)Alkylthio-, ($C_1$-$C_6$)Alkoxy-($C_2$-$C_4$)-alkoxy, Halogen-($C_1$ oder $C_1$)alkoxy- oder Methoxyethoxyethoxygruppe substituiert sein kann; ($C_3$-$C_6$)Alkenyl, ($C_3$-$C_6$) Alkinyl, ($C_5$ oder $C_6$)Cycloalkyl, ($C_1$-$C_4$)Alkoxycarbonyl-($C_1$ oder $C_2$)alkyl oder die Gruppe $-N{=}C[(C_1\text{-}C_4)\text{-Alkyl}]_2$ bedeuten, durch Umsetzung von Verbindungen der Formel II

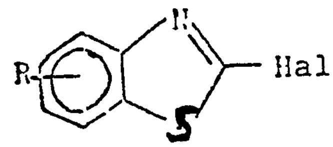

II

mit Verbindungen der Formel III,

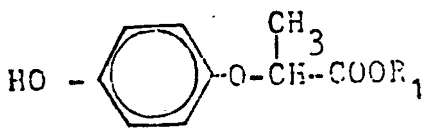

III

in Gegenwart von inerten unpolaren Lösungsmitteln und säurebindenden Mitteln, dadurch gekennzeichnet, daß die Reaktion in Anwesenheit eines Katalysators aus der Gruppe der quartären Ammonium- und Phosphoniumbasen, der Polyalkylenglycole oder deren Niederalkylether durchgeführt wird.

2. Verfahren nach Anspruch 1, dadurch gekennzeichnet, daß man als Katalysator ein quartäres Ammonium- oder Phosphoniumhalogenid verwendet.

3. Verfahren nach Anspruch 1, dadurch gekennzeichnet, daß man als Katalysatoren Polyethylene- oder Polypropylenglycole oder deren Niederalkylether vom Molekulargewicht 400 - 6000 verwendet.

4. Verfahren nach Anspruch 3, dadurch gekennzeichnet, daß man als Katalysatoren Polyethylene- oder Polypropylenglycole oder deren Niederalkylether vom Molekulargewicht 1000 - 3000 verwendet.

5. Verfahren nach Anspruch 1 - 3, dadurch gekennzeichnet, daß die Katalysatormenge 0,05 - 1 mol % bezogen auf die Komponente II beträgt.

6. Verfahren nach Anspruch 1 - 4, dadurch gekennzeichnet, daß die Katalysatormenge 0,1 - 0,5 mol %, bezogen auf die Komponente II beträgt.

7. Verfahren nach Anspruch 6, dadurch gekennzeichnet, daß thermische Abspaltung aus dem säurebindenden Mittel gebildete Wasser während der Reaktion azeotrop oder unter vermindertem Druck oder mit Hilfe eines Inertgasstromes abdestilliert wird.

**Claims**

1. A process for the preparation of a compound of the formula I,

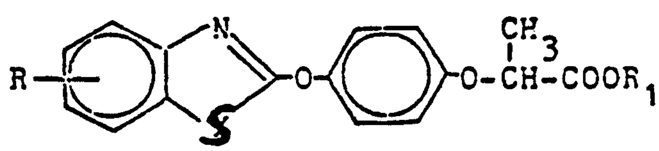

in which

R denotes halogen or CF3, and

$R_1$ denotes $(C_1$-$C_{12})$alkyl which may be substituted by one or two halogen atoms or by one $(C_1$-$C_6)$alkoxy, $(C_1$-$C_6)$alkylthio, $(C_1$-$C_6)$alkoxy$(C_2$-$C_4)$alkoxy, halo($C_1$ or $C_2$)alkoxy or methoxyethoxyethoxy group; $(C_3$-$C_6)$alkenyl, $(C_3$-$C_6)$alkynyl, ($C_5$ or $C_6$)cycloalkyl, $(C_1$-$C_4)$alkoxycarbonyl($C_1$ or $C_2$)alkyl or the $-N{=}C[(C_1$-$C_4)alkyl]_2$ group, by reacting a compound of the formula II

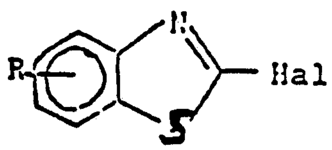

II

with a compound of the formula III

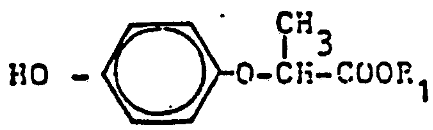

III

in the presence of inert, nonpolar solvents and acid binders, wherein the reaction is carried out in the presence of a catalyst from the series comprising the quaternary ammonium and phosphonium bases, the polyalkylene glycols or the lower alkyl ethers thereof.

2. The process as claimed in claim 1, wherein the catalyst used is a quaternary ammonium or phosphonium halide.

3. The process as claimed in claim 1, wherein the catalyst used is a polyethylene glycol or a polypropylene glycol or a lower alkyl ether thereof, of molecular weight 400 - 6,000.

4. The process as claimed in claim 3, wherein the catalyst used is a polyethylene glycol or a polypropylene glycol or a lower alkyl ether thereof, of molecular weight 1,000 - 3,000.

5. The process as claimed in any of claims 1 - 3, wherein the amount of catalyst is 0.05 - 1 mol %, relative to component II.

6. The process as claimed in any of claims 1 - 4, wherein the amount of catalyst is 0.1 - 0.5 mol %, relative to component II.

7. The process as claimed in claim 6, wherein the water formed from the acid binder by thermal cleavage is removed by distillation during the reaction azeotropically or under reduced pressure or with the aid of a stream of inert gas.

**Revendications**

1. Procédé pour préparer des composés répondant à la formule I:

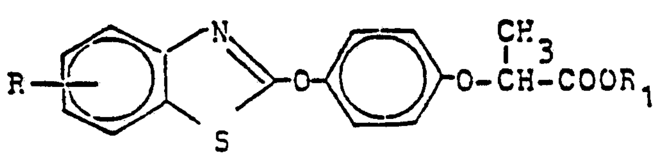

(I)

dans laquelle:

R représente un halogène ou $-CF_3$ et

$R_1$ représente un alkyle en $C_1$-$C_{12}$, éventuellement porteur d'un ou de deux atomes d'halogène ou d'un radical alcoxy en $C_1$-$C_6$, alkylthio en $C_1$-$C_6$, ($C_1$-$C_6$-alcoxy)-($C_2$-$C_4$-alcoxy), halogénoalcoxy en $C_1$ ou $C_2$ ou méthoxyéthoxyéthoxy; un alcényle en $C_3$-$C_6$, un alcynyle en $C_3$-$C_6$, un cycloalkyle en $C_5$ ou $C_6$, un ($C_1$-$C_4$-alcoxy)-carbonyl-($C_1$ ou $C_2$-alkyle) ou un radical $-N=C(alkyl)_2$ dont chacun des alkyles contient de 1 à 4 atomes de carbone,

par réaction de composés de formule II:

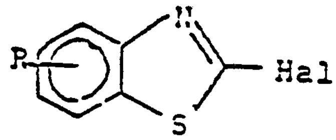

(II)

avec des composés de formule III:

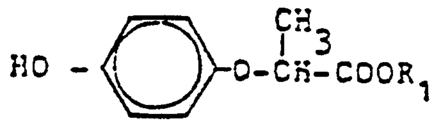

(III)

en présence de solvants inertes non polaires et d'accepteurs d'acides, procédé caractérisé en ce qu'on effectue la réaction en présence d'un catalyseur pris dans l'ensemble constitué par les bases d'ammoniums et de phosphoniums quaternaires, les poly-alkylène-glycols et les éthers alkyliques inférieurs de ces derniers.

2. Procédé selon la revendication 1 caractérisé en ce qu'on utilise, comme catalyseur, un halogénure d'ammonium ou de phosphonium quaternaire.

3. Procédé selon la revendication 1 caractérisé en ce qu'on utilise, comme catalyseurs, des polyéthylène-glycols ou poly-propylène-glycols ou leurs éthers alkyliques inférieurs qui ont des masses moléculaires de 400 à 6 000.

4. Procédé selon la revendication 3 caractérisé en ce qu'on utilise, comme catalyseurs, des polyéthylène-glycols ou poly-propylène-glycols ou leurs éthers alkyliques inférieurs qui ont des masses moléculaires de 1 000 à 3 000.

5. Procédé selon l'une quelconque des revendications 1 à 3, caractérisé en ce que la quantité du catalyseur est comprise entre 0,05 et 1 % en moles par rapport à la composante (II).

6. Procédé selon l'une quelconque des revendications 1 à 4, caractérisé en ce que la quantité du catalyseur est comprise entre 0,1 et 0,5 % en moles par rapport à la composante (II).

7. Procédé selon la revendication 6, caractérisé en ce que l'eau engendrée par décomposition thermique à partir de l'accepteur d'acides est éliminée au cours de la réaction par distillation azéotropique ou par distillation sous pression réduite ou au moyen d'un courant de gaz inerte.